(19) Europäisches Patentamt
European Patent Office
Office européen des brevets

(11) Veröffentlichungsnummer: **0 043 521**
**B1**

(12) **EUROPÄISCHE PATENTSCHRIFT**

(45) Veröffentlichungstag der Patentschrift: **16.05.84**

(51) Int. Cl.³: **C 07 C 45/00,** C 07 C 45/45, C 07 C 49/04

(21) Anmeldenummer: **81104967.5**

(22) Anmeldetag: **26.06.81**

(54) **Verfahren zur Herstellung von Pinakolin.**

(30) Priorität: **08.07.80 DE 3025777**

(43) Veröffentlichungstag der Anmeldung: **13.01.82 Patentblatt 82/2**

(45) Bekanntmachung des Hinweises auf die Patenterteilung: **16.05.84 Patentblatt 84/20**

(84) Benannte Vertragsstaaten: **BE CH DE FR GB IT LI NL**

(56) Entgegenhaltungen:
**DE - A - 2 918 521**
**FR - A - 2 364 876**

**CHEMICAL ABSTRACTS, Band 91, Heft 23, 3. Dezember 1979, Seite 599, Zusammenfassung 192827y, COLUMBUS, OHIO (US)**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Berendes, Otto, Dr., Lahnstrasse 5, D-4047 Dormagen 4 (DE)**
Erfinder: **Siegle, Peter, Dr., Am Reinholdsberg 1, D-5000 Köln 80 (DE)**

EP 0 043 521 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Pinakolin

Die vorliegende Erfindung betrifft ein neues Verfahren zur Herstellung von Pinakolin (2,2-Dimethyl-3-oxo-butan).

Es ist bereits bekanntgeworden, daß man Pinakolin erhält, wenn man 2-Methyl-but-2-en (oder 2-Methyl-but-1-en) unter bestimmten Bedingungen in wäßrigem Medium in Gegenwart einer starken anorganischen Säure (mindestens 15 Gewichtsprozent Säure) mit Formaldehyd umsetzt (vgl. DE-PS 24 61 503). Ferner ist eine Modifizierung dieses Verfahrens bekanntgeworden, welche dadurch gekennzeichnet ist, daß die Umsetzung unter Zugabe eines Salzes einer starken anorganischen Säure, das mindestens teilweise in dem Reaktionssystem löslich ist, erfolgt; durch diese Maßnahme soll es möglich sein, die sonst erforderliche Säurekonzentration und -menge zu verringern (vgl. DE-OS 29 18 521).

Das für die beiden genannten Verfahren als Ausgangsprodukt benötigte 2-Methylbuten muß bei großtechnischer Durchführung aus einer $C_5$-Benzinfraktion durch Anlagerung von Alkoholen (wie z. B. Methanol), wobei sich t-Amylether (wie z. B. t-Amylmethylether) bilden, isoliert werden. In einem zweiten Reaktionsschritt müssen die tertiären Ether dann unter Wiederabspaltung des Alkohols in 2-Methyl-buten rückverwandelt werden.

Es ist außerdem bekannt, daß man Pinakolin durch eine PRINS-Reaktion erhalten kann, indem man 2-Hydroxy-2-methylbutan in wäßrigem Medium in Gegenwart einer starken anorganischen Säure mit Formaldehyd bei Temperaturen zwischen 92 und 100°C umsetzt (vgl. »Chemical Abstracts«, Band 91 (1979), Zusammenfassung Nr.192 827 y). Ein sehr wesentlicher, technischer Nachteil dieses Verfahrens besteht darin, daß der als Ausgangsprodukt benötigte tertiäre Alkohol 2-Hydroxy-2-methylbutan nicht großtechnisch verfügbar ist, so daß das Verfahren für eine Anwendung in großtechnischem Maßstab nicht in Frage kommt.

Es wurde nun überraschend gefunden, daß man Pinakolin der Formel

$$(CH_3)_3C - CO - CH_3 \qquad (I)$$

in guter Ausbeute und reiner Form erhält, wenn man ein 2-Alkoxy-2-methylbutan der allgemeinen Formel

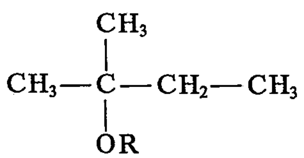

(II)

worin R für einen $C_{1-4}$-Alkylrest steht,
in wäßrigem Medium in Gegenwart einer starken anorganischen Säure mit Formaldehyd bei Temperaturen zwischen 50 und 90°C umsetzt.

Dadurch, daß die tertiären Ether (II), d. h. die Alkohol-/2-Methylbuten-Anlagerungsprodukte, erfindungsgemäß direkt für die Pinakolin-Synthese eingesetzt werden können, wird gegenüber den vorbekannten, von 2-Methylbuten ausgehenden Verfahren ein Reaktionsschritt eingespart. Der entscheidende technische Vorteil des erfindungsgemäßen Verfahrens gegenüber dem ebenfalls vorbekannten, von 2-Hydroxy-2-methylbutan ausgehenden Verfahren liegt darin, daß das nach der Erfindung als Ausgangsprodukt bevorzugt verwendete 2-Methoxy-2-methylbutan (t-Amylmethylether) — im Gegensatz zu 2-Hydroxy-2-methylbutan — großtechnisch verfügbar ist, da es in beträchtlichen Mengen als Antiklopfmittel verwendet wird, ebenso wie der niedriger homologe t-Butylmethylether; hierdurch bietet das erfindungsgemäße Verfahren die Möglichkeit einer großtechnischen Anwendung.

Es ist allgemein bekannt, daß tertiäre Alkohole wie 2-Hydroxy-2-methylbutan mit Säuren leicht reagieren (wobei Dehydratisierung und ggf. Umlagerungen eintreten). Dies gilt auch für die vorbekannte Umsetzung eines tertiären Alkohols mit Formaldehyd und Säure (unter den allgemeinen Bedingungen der PRINS-Reaktion.

Überraschend ist jedoch, daß gemäß der Erfindung einfache Alkylether (insbesondere der Methylether des 2-Hydroxy-2-methylbutans) unter den vergleichsweise milden Reaktionsbedingungen praktisch genauso leicht reagieren wie der freie tertiäre Alkohol, wobei die Reaktionstemperatur sogar deutlich niedriger liegt (50—90°C) als bei dem vorbekannten Verfahren (92—100°C).

Es ist bekannt, daß Methylether üblicherweise gerade dann als Schutzgruppen verwendet werden, wenn sie Methoxygruppe erhalten und nicht wieder rückgespalten werden soll. Methylether gelten als zu stabil, um routinemäßig als Schutzgruppen für Alkoholfunktionen verwendet zu werden. Solche Methylether können nur unter schärferen Reaktionsbedingungen, z. B. durch Behandeln mit $BCl_3$, gespalten werden (vgl. z. B. J. F. W. McOmie, Protective Groups in Organic Chemistry, 1973, S. 96—97). Dementsprechend findet auch unter den Reaktionsbedingungen des erfindungsgemäßen Verfahrens keine Etherspaltung statt.

Überraschend ist außerdem, daß — wie eigene Versuche gezeigt haben — unter jeweils vergleichbaren Reaktionsbedingungen zwar einerseits 2-Hydroxy- und 2-Methoxy-2-methylbutan praktisch gleich gut (d. h. gleich schnell mit fast gleich hohen Ausbeuten) zu Pinakolin reagieren, daß aber andererseits bei dem jeweils isomeren Verbindungspaar, 3-Hydroxy- und 3-Methoxy-2-methylbutan, der Ether nur mit äußerst geringer Ausbeute (3,3%) zu Pinakolin reagiert, während der Alkohol mit wesentlich höherer Ausbeute (32,4%) Pinakolin ergibt. Bei der glatten Umsetzung von 2-Methoxy-2-methylbutan handelt es sich somit um einen überraschenden Sonderfall.

Bei Verwendung von 2-Methoxy-2-methylbutan kann der Reaktionsablauf durch folgendes Formelschema wiedergegeben werden:

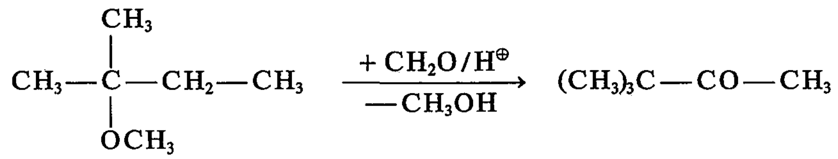


Die erfindungsgemäß verwendbaren 2-Alkoxy-2-methylbutane (II) sind bekannt. Sie können, wie bereits angegeben, hergestellt werden durch Anlagerung der entsprechenden Alkohole an 2-Methyl-but-2-en oder 2-Methyl-but-1-en bzw. an Gemische der beiden Isomeren, großtechnisch durch Alkohol-Anlagerung an $C_5$-Benzinfraktionen, welche neben anderen Kohlenwasserstoffen auch die genannten Pentene enthalten.

Als Beispiele seien 2-Methoxy-2-methylbutan (t-Amylmethylether), 2-Ethoxy-2-methylbutan (t-Amylethylether) und 2-Propoxy-2-methylbutan (t-Amyl-propylether) genannt.

Der für die erfindungsgemäße Umsetzung benötigte Formaldehyd kann in allen handelsüblichen Formen und Konzentrationen verwendet werden, also zum Beispiel als wäßrige Lösung von 5—70%, vorzugsweise von 20—60%; aber man kann auch Paraformaldehyd einsetzen.

Die Umsetzung wird, wie oben angegeben, bei Temperaturen zwischen 50 und 90° C durchgeführt.

Bei der Durchführung des erfindungsgemäßen Verfahrens werden auf 1 Mol 2-Alkoxy-2-methylbutan (II) 1—1,5 Mol, vorzugsweise 1—1,2 Mol Formaldehyd eingesetzt.

Als starke anorganische Säuren können insbesondere Salzsäure, Bromwasserstoffsäure und Schwefelsäure eingesetzt werden. Die Menge der für die erfindungsgemäße Umsetzung benötigten anorganischen Säure und ihre Konzentration in der wäßrigen Phase hängen von der Natur der Säure ab. Verwendet man Salzsäure oder Bromwasserstoffsäure, so soll deren Konzentration 10—40%, vorzugsweise 25—40% betragen. Verwendet man Schwefelsäure, so soll die Konzentration 20—60%, mindestens jedoch ebenfalls 10% betragen.

Die Reaktionskomponenten tert. Ether (II) und Formaldehyd können gleichzeitig oder nacheinander der Säure zugesetzt werden. Es hat sich als besonders zweckmäßig erwiesen, die wäßrige Säurelösung vorzulegen und bei Raumtemperatur den tert. Ether (II) zuzugeben und anschließend bei erhöhter Temperatur den Formaldehyd, im allgemeinen in Form einer wäßrigen Formalinlösung, langsam zutropfen zu lassen. Eine anschließende Nachreaktionszeit, ebenfalls bei erhöhter Temperatur, von mehreren Stunden (0,5—10 Stunden) ist ratsam, bevor der Ansatz aufgearbeitet wird.

Weiterhin ist es für die erfindungsgemäße Umsetzung wesentlich, daß während der Reaktion und der Nachreaktion für eine intensive Durchmischung des Reaktionsgemisches gesorgt wird. Dies kann durch Verwendung geeigneter Rührer und entsprechender Drehzahlen der Rührer, gegebenenfalls auch durch Zugabe kleiner Mengen eines Emulgators zum Reaktionsgemisch erreicht werden.

Nach Beendigung der Reaktion wird das Reaktionsgemisch abgekühlt und mit Natronlauge neutral gestellt; die beiden Phasen werden getrennt. Die organische Phase enthält das Pinakolin, das man am zweckmäßigsten durch Destillation isoliert und reinigt.

Der bei der Umsetzung freiwerdende Alkohol kann bei Durchführung der Reaktion bei relativ niedrigen Temperaturen und niedrigen Säurekonzentrationen nach Neutralisation des Ansatzes aus der Wasserphase wiedergewonnen werden. Bei höheren Temperaturen und höheren Säurekonzentrationen von Salzsäure und Bromwasserstoffsäure wird der frei werdende Alkohol zum entsprechenden Alkylchlorid bzw. Alkylbromid umgesetzt; dieses findet sich bei der Aufarbeitung in der organischen Phase und kann destillativ zum Pinakolin getrennt werden.

Pinakolin kann als Lösungsmittel, ferner als Zwischenprodukt in der organischen Synthese, beispielsweise zur Herstellung von bekannten herbiziden Wirkstoffen verwendet werden. Als Beispiel sei die Synthese der herbizid besonders wirksamen Verbindung 4-Amino-6-tert.-butyl-3-methylthio-1,2,4-triazin-5(4H)-on, ausgehend von Pinakolin, genannt (vgl. DE-AS 26 48 300; DE-PS 24 61 503; DE-PS 17 95 784).

## Herstellungsbeispiele

### Beispiel 1

Zu 600 ml 38%iger Salzsäure wurden bei Raumtemperatur unter Rühren innerhalb von 30 Minuten

255 g (2,5 Mol) 2-Methoxy-2-methylbutan zugegeben. Anschließend wurden bei 70° C innerhalb von 2 Stunden 250 g 30%ige wäßrige Formalinlösung (2,5 Mol Formaldehyd) zugetropft. Nach beendetem Zulauf ließ man noch 2 Stunden bei 80° C nachreagieren, dann wurde das Reaktionsgemisch abgekühlt und mit Natronlauge neutral gestellt; die organische Phase wurde abgetrennt, getrocknet und destilliert.

Ausbeute: 165 g (66% d. Th.) reines Pinakolin, Siedepunkt: 106—107° C.

In den dem Destillationskühler nachgeschalteten Kühlfallen wurden 70 g (55% d. Th.) Methylchlorid kondensiert.

Beispiel 2

Zu 120 ml 38%iger Salzsäure wurden bei Raumtemperatur innerhalb von 10 Minuten unter Rühren 58,5 g (0,5 Mol) 2-Ethoxy-2-methylbutan zugegeben. Anschließend wurden bei 70° C 52,5 g 30%ige wäßrige Formalinlösung (0,525 Mol Formaldehyd) zugetropft. Die Nachreaktion und Aufarbeitung erfolgte wie bei Beispiel 1.

Ausbeute: 30,5 g (61% d. Th.) reines Pinakolin.

Beispiel 3

Zu 120 ml 39%iger Bromwasserstoffsäure wurden bei Raumtemperatur unter Rühren 51 g (0,5 Mol) 2-Methoxy-2-methylbutan gegeben. Dann ließ man bei 70° C 52,5 g 30%ige wäßrige Formalinlösung (0,525 Mol $CH_2O$) zutropfen. Nach 2stündiger Nachreaktion bei 80° C wurde aufgearbeitet, wie in Beispiel 1 beschrieben.

Ausbeute: 21 g (42% d. Th.) reines Pinakolin.

Beispiel 4

Zu 240 ml 30%iger Schwefelsäure wurden bei Raumtemperatur unter Rühren 102 g (1 Mol) 2-Methoxy-2-methylbutan gegeben. Innerhalb von 3 Stunden wurden anschließend bei 54° C 110 g 30%ige wäßrige Formalinlösung (1,1 Mol $CH_2O$) zugetropft. Nach 5stündiger Nachreaktion bei 70° C wurde wie zuvor beschrieben aufgearbeitet.

Ausbeute: 45 g (45% d. Th.) reines Pinakolin.

Aus der wäßrigen Phase konnten 27,2 g (85% d. Th.) Methanol isoliert werden.

**Patentansprüche**

1. Verfahren zur Herstellung von Pinakolin, $(CH_3)_3C-CO-CH_3$ (I), dadurch gekennzeichnet, daß man ein 2-Alkoxy-2-methylbutan der allgemeinen Formel

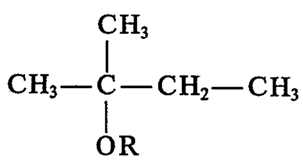

(II)

worin R für einen $C_{1-4}$-Alkylrest steht,
in wäßrigem Medium in Gegenwart einer starken anorganischen Säure mit Formaldehyd bei Temperaturen zwischen 50 und 90° C umsetzt.

2. Verfahren nach Anspruch 1, dadurch gekennzeichnet, daß man auf 1 Mol 2-Alkoxy-2-methylbutan (II) 1—1,5 Mol, vorzugsweise 1—1,2 Mol, Formaldehyd einsetzt.

3. Verfahren nach Anspruch 1, dadurch gekennzeichnet, daß man als starke anorganische Säure Chlorwasserstoffsäure, Bromwasserstoffsäure oder Schwefelsäure einsetzt.

**Claims**

1. Process for the preparation of pinacolone, $(CH_3)_3C-CO-CH_3$ (I), characterised in that a 2-alkoxy-2-methylbutane of the general formula

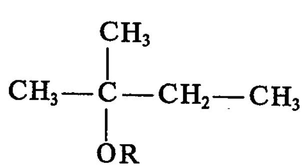

(II)

wherein R represents a $C_{1-4}$-alkyl radical,
is reacted with formaldehyde at temperatures between 50 and 90° C in an aqueous medium in the presence of a strong inorganic acid.

2. Process according to claim 1, characterised in that 1—1,5 moles, preferably 1—1,2 moles, of formaldehyde are employed per mole of 2-alkoxy-2-methylbutane (II).

3. Process according to claim 1, characterised in that hydrochloric acid, hydrobromic acid or sulphuric acid is employed as the strong inorganic acid.

**Revendications**

1. Procédé pour la fabrication de pinacolone $(CH_3)_3C — CO — CH_3$, caractérisé en ce que l'on fait réagir un 2-alcoxy-2-méthylbutane de formule générale

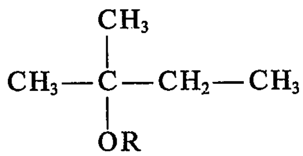

(II)

dans laquelle R représente un reste alkyle en $C_1 — C_4$,
avec le formaladéhyde en milieu aqueux, en présence d'un acide inorganique fort, à des températures comprises entre 50 et 90° C.

2. Procédé selon la revendication 1, caractérisé en ce que l'on utilise 1—1,5 mole, de préférence 1—1,2 mole, de formaldéhyde pour 1 mole de 2-alcoxy-2-méthylbutane (II).

3. Procédé selon la revendication 1, caractérisé en ce que l'on utilise comme acide inorganique fort l'acide chlorhydrique, l'acide bromhydrique ou l'acide sulfurique.